# EUROPÄISCHE PATENTANMELDUNG

(11) **EP 2 745 822 A1**
(43) Veröffentlichungstag der Anmeldung: **25.06.2014**
(21) Anmeldenummer: 14160632.7
(22) Anmeldetag: 09.06.2011
(51) Int. Cl.: A61F 13/02, A61L 15/20, A61L 15/34, A61M 1/00

(54) **Wundauflage, enthaltend Vliesstoff und Salbengrundlage, zur Unterdrucktherapie**

(62) Teilanmeldung aus: 11004725.5
(71) Anmelder: Paul Hartmann AG, 89522 Heidenheim (DE)
(72) Erfinder: Croizat, Pierre, 89542 Herbrechtingen (DE); Eckstein, Axel, 89522 Heidenheim (DE)
(74) Vertreter: Aechter, Bernd

(57) **Zusammenfassung**

Die Erfindung betrifft eine Vorrichtung zur Unterdrucktherapie von Wunden umfassend, (a) ein Abdeckmaterial zum luftdichten Verschließen eines Wundraums; (b) gegebenenfalls ein Mittel zum Anschließen einer Unterdruckquelle; und (c) eine Wundauflage, enthaltend (c1) einen Vliesstoff, der mit (c2) einer Salbengrundlage benetzt ist, sowie ein Verfahren zur Herstellung einer entsprechenden Wundauflage. Ferner betrifft die Erfindung die Verwendung eines Vliesstoffs, der mit einer Salbengrundlage benetzt ist, zur Anwendung als Wundauflage in der Unterdrucktherapie von Wunden.

## Beschreibung

Die Erfindung betrifft eine Vorrichtung zur Unterdrucktherapie von Wunden umfassend, (a) ein Abdeckmaterial zum luftdichten Verschließen eines Wundraums; (b) gegebenenfalls ein Mittel zum Anschließen einer Unterdruckquelle; und (c) eine Wundauflage, enthaltend (c1) einen Vliesstoff und (c2) eine Salbengrundlage, sowie ein Verfahren zur Herstellung einer entsprechenden Wundauflage. Ferner betrifft die Erfindung die Verwendung eines Vliesstoffs, der mit einer Salbengrundlage benetzt ist, zur Anwendung als Wundauflage in der Unterdrucktherapie von Wunden.

Unter einer Wunde wird die Trennung des Zusammenhangs von Geweben der Körperhülle bei Menschen oder Tieren verstanden. Sie kann mit einem Verlust an Substanz verbunden sein.

Vorrichtungen zur Unterdrucktherapie von Wunden sind im Stand der Technik bekannt.

So beschreibt beispielsweise die WO 1993/009727 A1 eine Vorrichtung zur Förderung der Wundheilung durch die Applikation von Unterdruck auf dem die Wunde aufweisenden und die Wunde umgebenden Hautbereich. Die Vorrichtung gemäß WO 1993/009727 A1 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung.

Geräte zur Unterdrucktherapie von Wunden sind kommerziell erhältlich, beispielsweise das VivanoTec^{®} der Firma Paul Hartmann AG. Bei kommerziell erhältlichen Geräten wird oftmals eine Wundauflage eingesetzt, welche einen offenzelligen Polymer-Schaumstoff, wie beispielsweise Polyvinylalkohol (PVA) oder Polyurethan (PU), enthält.

Die kommerziell üblichen Schaumauflagen werden, abhängig vom angelegten Unterdruck, unterschiedlich stark komprimiert. Dadurch kann eine Verengung der für den Abtransport des Wundexsudats nötigen Durchgänge entstehen. Weiterhin kann eine Verklebung des Schaums mit dem Wundgrund auftreten. Neu gebildetes Gewebe kann in den Schaum einwachsen. Dieses Problem ist eine bekannte Komplikation bei der Unterdrucktherapie von Wunden (FDA complaint database). Um dieses Problem zu lösen, werden häufig zusätzliche Wundkontaktschichten zwischen Schaum und Wundgrund eingebracht, beispielsweise eine Folie (siehe z.B. WO 2001/85248 A1). Diese zusätzlichen Wundkontaktschichten können aber den Durchfluss von Exsudat vermindern.

Verklebter Schaum muss zudem beim Wechseln des Verbands aufwendig entfernt werden, zum Beispiel durch Spülen mit Ringerlösung. In den Schaum eingewachsenes Gewebe kann beim Verbandwechsel zu einer Gewebetraumatisierung und damit zur Störung der Wundheilung führen.

Beim Einsatz herkömmlicher Wundauflagen können zudem Schaumstoffpartikel in die Wunde gelangen. Dieses Problem wird noch verstärkt, wenn die Wundauflage vor der Anwendung auf die Größe der Wunde zugeschnitten wird, da dann insbesondere lose Schaumstoffpartikel an den Schnitträndern erzeugt werden. Auch hat sich gezeigt, dass die üblichen Polymer-Schaumstoffe teilweise mit der Abdeckfolie verkleben. Ein Verkleben der Wundauflage mit der Abdeckfolie wirkt sich insbesondere beim Entfernen des Verbandes nachteilig aus.

Der vorliegenden Erfindung liegt die Aufgabe zugrunde, die Unterdruckbehandlung von Wunden weiter zu verbessern und die Nachteile des Standes der Technik zu überwinden. Es sollen mit der vorliegenden Erfindung Vorrichtungen und Verfahren zur Unterdruckbehandlung von Wunden zur Verfügung gestellt werden, mit denen eine Behandlung möglichst wirksam und möglichst schonend durchgeführt werden kann.

Insbesondere soll die vorliegende Erfindung eine Unterdruckbehandlung von Wunden ermöglichen, die beim Patienten einen Wechsel der Wundauflagen in Intervallen von beispielsweise bis zu drei Tagen ermöglicht. Während eines Intervalls soll die Wundauflage vom Patienten subjektiv als angenehm empfunden werden. Druckreiz und Hautrötungen sollen vermieden werden. Beim Wechsel der Wundauflage nach einem Zeitraum von beispielsweise bis zu 3 Tagen sollen möglichst wenig unangenehme Gerüche auftreten. Die Keimdichte in der ausgewechselten Wundauflage soll niedrig sein.

Es soll eine Wundauflage bereitgestellt werden, bei der ein Verkleben mit der Abdeckfolie weitgehend vermieden wird. Die Saugkraft soll nicht in Abhängigkeit vom Abstand zum Port abnehmen. Weiterhin soll eine Wundauflage bereitgestellt werden, bei der das Anlegen eines geringen Unterdrucks ausreicht.

Die Aufgaben konnten unerwartet durch die Verwendung einer Wundauflage, die einen Vliesstoff und eine Salbengrundlage enthält, gelöst werden. Überraschend wurde ebenfalls gefunden, dass eine Vorrichtung zur Unterdrucktherapie umfassend mindestens eine Wundauflage, die mit einer Salbengrundlage benetzt ist, zu einer vorteilhaften, d.h. sehr wirksamen und sehr schonenden Behandlung von Wunden besonders geeignet ist. Durch die Verwendung der erfindungsgemäßen Wundauflage konnte eine bessere Anpassung an den Wundgrund erreicht werden. Weiterhin wurde der von den Patienten empfundene Druckreiz durch die Wundauflage vorteilhaft verringert.

Es hat sich herausgestellt, dass die Aufgaben insbesondere vorteilhaft gelöst werden, wenn ein spezieller Vliesstoff und/oder eine spezielle Salbengrundlage verwendet werden und/oder wenn Art des Vliesstoffs und Art und Menge der Salbengrundlage gegenseitig aufeinander abgestimmt werden. Hierdurch konnte insbesondere eine deutliche Verringerung der Anzahl von Partikeln, die beim Schneiden entstehen, und eine vorteilhafte Verringerung des Verklebens des Wundgrunds mit der Wundauflage erreicht werden. Weiterhin hat sich gezeigt, dass bei der Verwendung der erfindungsgemäßen Wundauflage im Gegensatz zu den Wundauflagen aus dem Stand der Technik ein geringerer Unterdruck notwendig ist, was vom Patienten üblicherweise als angenehm empfunden wird.

Gegenstand eines ersten Aspekts der Erfindung ist daher eine Vorrichtung zur Unterdrucktherapie von Wunden, umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen Vliesstoff, der mit
(c2) einer Salbengrundlage benetzt ist, die bevorzugt einen Tropfpunkt von 20 bis 80°C aufweist,
wobei der Anteil an Salbengrundlage (c2) 10 bis 95 Gew.-% beträgt, bezogen auf das Gesamtgewicht der Wundauflage.

Gegenstand eines zweiten Aspekts der Erfindung ist eine Vorrichtung zur Unterdrucktherapie von Wunden, umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen Vliesstoff, der mit
(c2) einer Salbengrundlage auf Basis eines Triglycerids benetzt ist.

Gegenstand eines dritten Aspekts der Erfindung ist eine Vorrichtung zur Unterdrucktherapie von Wunden, umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen speziellen Vliesstoff, der eine Luftdurchlässigkeit von 200 bis 5000 l/(m²sec), gemessen gemäß DIN EN ISO 9237 aufweist, und mit
(c2) einer Salbengrundlage benetzt ist.

Bevorzugt beträgt der Anteil an Salbengrundlage (c2) 10 bis 95 Gew.-%, bezogen auf das Gesamtgewicht der Wundauflage.

Ebenfalls umfasst die Erfindung beliebige Kombinationen der genannten Aspekte.

Ferner ist Gegenstand der Erfindung die Verwendung eines Vliesstoffs, der mit einer Salbengrundlage benetzt ist, bevorzugt mit einer Salbengrundlage mit Tropfpunkt von 20 bis 80°C, insbesondere mit einer Triglycerid-Salbengrundlage, als Wundauflage zur oder in der Unterdrucktherapie von Wunden. Das heißt, Gegenstand der Erfindung ist ein Vliesstoff, der mit einer Triglycerid-Salbengrundlage benetzt ist, als Wundauflage in der Unterdrucktherapie von Wunden.

Ebenfalls ein Gegenstand der Erfindung ist ein Verfahren zur Herstellung einer Wundauflage, umfassend die Schritte
(I) Erwärmen einer Salbengrundlage, bevorzugt über den Tropfpunkt;
(II) Einbringen des Vliesstoffs in die erwärmte Salbengrundlage, so dass der Vliesstoff mit der Salbengrundlage benetzt wird;
(III) gegebenenfalls temporäres Zusammendrücken des Vliesstoffs, bevorzugt auf mindestens 90 % und höchstens 10 % seines ursprünglichen Volumens, um eine Benetzung der Oberfläche des Vliesstoffs mit der Salbengrundlage zu erreichen; und
(IV) gegebenenfalls Entfernen der überschüssigen Salbengrundlage, bevorzugt durch Ausdrücken des Vliesstoffs.

Zudem sind Wundauflagen, erhältlich durch das erfindungsgemäße Verfahren, Gegenstand der Erfindung.

Ein weiterer Gegenstand der Erfindung ist die Verwendung eines Vliesstoffs (c1), der mit einer Salbengrundlage (c2) benetzt ist, als Wundauflage (c) zur oder in der Unterdrucktherapie von Wunden, insbesondere von Brandwunden, um durch mechanische Traumatisierung entstandene Wunden, um durch Einwirkung von Chemikalien entstandene Wunden, um durch Stoffwechselstörung verursachte Wunden um durch Durchblutungsstörung verursachte Wunden oder durch Druckgeschwüre verursachte Wunden.

Die neue erfindungsgemäße Vorrichtung beziehungsweise die erfindungsgemäße Verwendung der Wundauflage zeichnet sich durch mehrere unerwartete Vorteile aus. Durch die Benetzung des Vliesstoffs mit der Salbengrundlage konnte die Anzahl der Partikel, welche unerwünscht in die Wunde gelangen, vorteilhaft reduziert werden.

Die Verwendung der erfindungsgemäßen Wundauflage verbesserte die atraumatischen Eigenschaften, sodass eine Unterdruckbehandlung gegebenenfalls auch ohne zusätzliche Wundkontaktschichten ermöglicht wurde.

Durch die Benetzung des Vliesstoffs mit der Salbengrundlage verringerte sich bei den Patienten das subjektive Gefühl des Juckreizes und der empfundene Härte des Vliesstoffs während der Unterdrucktherapie. Der Vliesstoff fühlte sich angenehmer an und die Patientencompliance (Befolgung der Therapieanweisung durch den Patienten) wurde erhöht.

Weiterhin hat sich gezeigt, dass trotz Einsatzes der Salbengrundlage die Saugkraft in Abhängigkeit vom Port nicht unerwünscht abnimmt. Überraschenderweise kann mit der erfindungsgemäßen Wundauflage sowohl eine ausreichende Durchleitung von Wundexsudat als auch ein die Wundheilung unterstützender Effekt erreicht werden, insbesondere bei Einsatz einer Salbengrundlage, welche Triglyceride und gegebenenfalls Diglyceride umfasst. Dies könnte auf der Aktivität der endogen in der Wunde vorhandenen Lipoproteinlipase beruhen. Die aus den Triglyceriden der Salbengrundlage freigesetzten Fettsäuren scheinen sich positiv auf die Wundheilung auszuwirken.

Ein die Wundheilung unterstützender Effekt kann bei der erfindungsgemäßen Wundauflage insbesondere dann erreicht werden, wenn durch die Wundauflage der Wunde eine ausreichende Menge an Salbengrundlage bereitgestellt wird. Eine zu große Menge an diesen Komponenten kann jedoch zu einer Verstopfung des Vliesstoffs führen und somit die Durchleitung von Wundexsudat behindern. Bevorzugt beträgt der Anteil an Salbengrundlage 10 bis 95 Gew.-%, mehr bevorzugt 30 bis 85 Gew.-%, noch mehr bevorzugt 35 bis 80 Gew.-%, besonders bevorzugt 40 bis 75 Gew.-%, insbesondere 45 bis 70 Gew.-%, bezogen auf das Gesamtgewicht der Wundauflage.

Das erfindungsgemäße Herstellungsverfahren ist technisch einfach und kann auch bei als Fertigprodukt bezogenen (medizinischen) Vliesstoffen eingesetzt werden. Es erfolgt eine Benetzung, wodurch in einem überwiegenden Teil der Vliesstoffbereiche die Freisetzung von Partikeln reduziert wird, wenn der Vliesstoff geschnitten wird. Im Gegensatz zu dem aus dem Stand der Technik bekannten Verfahren, bei welchem das Imprägnierungsmittel vor dem Aushärten beziehungsweise der Synthese des Schaums zugegeben wird (siehe EP 0 335 669 B1), ergeben sich keine unerwünschten Wechselwirkungen zwischen Salbengrundlage auf der einen Seite und dem Vliesstoff auf der anderen Seite.

Ein weiterer Vorteil der erfindungsgemäßen Wundauflage besteht darin, dass ein Verkleben und/oder Verwachsen des Wundgrundes mit der Wundauflage selbst über einen Zeitraum von beispielsweise bis zu 3 Tagen weitgehend vermieden werden kann. Eine Traumatisierung der Wunde beim Verbandswechsel kann vermieden werden. Dies erhöht die Wirksamkeit der Wundbehandlung. Somit wird beim Patienten ein Wechsel der Wundauflagen in Intervallen von beispielsweise bis zu drei Tagen ermöglicht. Während des Intervalls von drei Tagen wurde die erfindungsgemäße Wundauflage vom Patienten als angenehm empfunden. Druckreiz, Juckreiz und Hautrötungen wurden in der Regel vermieden. Beim Wechsel der Wundauflage nach einem Zeitraum von 3 Tagen traten wenig unangenehme Gerüche auf. Die Keimdichte in der ausgewechselten Wundauflage war unerwartet niedrig.

Die Bestandteile (a) bis (c) der erfindungsgemäßen Vorrichtung werden nachstehend beschrieben.

Die erfindungsgemäße Vorrichtung umfasst ein Abdeckmaterial (a) zum luftdichten Verschließen des Wundraums. Unter Wundraum wird die Wunde und gegebenenfalls die anliegende Wundumgebung verstanden. Unter "luftdichtem Verschließen" soll hier nicht verstanden werden, dass keinerlei Gasaustausch zwischen dem Wundraum und seiner Umgebung stattfindet. Vielmehr ist mit "luftdichtem Verschließen" in diesem Zusammenhang gemeint, dass unter Berücksichtigung der eingesetzten Unterdruckpumpe der für die Unterdrucktherapie von Wunden nötige Unterdruck aufrecht erhalten werden kann. Es können deshalb auch Abdeckmaterialien eingesetzt werden, welche eine geringfügige Gaspermeabilität aufweisen, solange der für die Unterdrucktherapie notwendige Unterdruck aufrechterhalten werden kann.

In einer bevorzugten Ausführungsform der Erfindung umfasst das Abdeckmaterial zum luftdichten Verschließen der Wunde ein wasserunlösliches Polymer oder eine Metallfolie. Das Abdeckmaterial weist bevorzugt eine Dicke von 10 µm bis 10.000 µm, insbesondere von 25 µm bis 100 µm, auf.

In einer bevorzugten Ausführungsform der Erfindung handelt es sich bei dem Abdeckmaterial (a) um ein wasserunlösliches Polymer. Bevorzugt weist das wasserunlösliche Polymer eine Löslichkeit von 10 mg/l oder weniger, mehr bevorzugt von 1 mg/ml oder weniger, insbesondere von 0,0001 bis 1 mg/ml, auf (bestimmt gemäß Säulenelutionsmethode nach EU-Richtlinie RL67-548-EWG, Anhang V Kap. A6). Beispiele sind Polyurethan, Polyester, Polypropylen, Polyethylen, Polyamid oder Polyvinylchlorid, Polyorganosiloxan (Silikon) oder eine Mischung daraus. Die genannten Polymere liegen hierbei bevorzugt in nicht-zellulärer Form vor.

Es hat sich gezeigt, dass durch ein Abdeckmaterial mit einer speziellen Wasserdampfdurchlässigkeit die eingangs erläuterten Aufgaben besonders vorteilhaft gelöst werden können. In einer bevorzugten Ausführungsform weist daher das Abdeckungsmaterial eine Wasserdampfdurchlässigkeit von 100 bis 2500 g/m² x 24h, mehr bevorzugt von 500 bis 2000 g/m² x 24h, noch mehr bevorzugt von 800 bis 1600 g/m² x 24h, insbesondere von 1050 bis 1450 g/m² x 24h, bestimmt gemäß DIN EN 13726-2 bei 23°C und 85 % relativer Luftfeuchte, auf. Insbesondere die Kombination einer Abdeckfolie (a) mit vorstehend genannter Wasserdampfdurchlässigkeit mit einem Vliesstoff (c1) mit nachstehend beschriebenen physikalischen Eigenschaften ist besonders vorteilhaft.

In einer bevorzugten Ausführungsform werden Abdeckmaterial und das Mittel zum Anschließen einer Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Die erfindungsgemäße Vorrichtung zur Unterdrucktherapie umfasst gegebenenfalls ein Mittel (b) zum Anschließen einer Unterdruckquelle, d.h. ein Mittel zur Herstellung von Unterdruck im Wundraum. In einer bevorzugten Ausführungsform handelt es sich hierbei um ein Mittel (b) zur funktionellen Verbindung des Wundraums mit einer außerhalb des Abdeckmaterials befindlichen Unterdruckquelle, sodass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und die Wunde gebildete Zwischenraum verstanden.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben. 1 mmHg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von mindestens 20 mm Hg und höchstens 250 mm Hg, bevorzugt von mindestens 50 mm Hg und höchstens 150 mm Hg. Dieser Unterdruckbereich hat sich als für die Wundheilung vorteilhaft erwiesen. In einer bevorzugten Ausführungsform der Erfindung handelt es sich bei dem Unterdruck um einen Unterdruck von mindestens 80 mm Hg und höchstens 140 mm Hg, mehr bevorzugt von mindestens 120 mm Hg und höchstens 130 mm Hg.

Die erfindungsgemäße Vorrichtung zur Unterdrucktherapie von Wunden umfasst, wie vorstehend geschildert, bevorzugt ein Mittel (b) zum Anschließen einer Unterdruckquelle, d.h. ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel (b) um eine Verbindungsleitung, bevorzugt einen Schlauch, insbesondere einen Silikon-Drainageschlauch. Die Verbindungsleitung kann durch das Abdeckmaterial hindurchgeführt werden. Alternativ kann die mindestens eine Verbindungsleitung unter dem Rand des Abdeckmaterials durchgeführt werden. In beiden Fällen ist die Durchtrittsstelle luftdicht abzudichten, damit der gewünschte Unterdruck im Verband aufrechterhalten werden kann. Als Abdichtmittel eignet sich beispielsweise eine Klebefolie, eine Klebemasse, oder ein Klebestreifen. Bei der Verbindungsleitung kann es sich beispielsweise um einen Schlauch, um ein Rohr oder um einen anderen Körper mit einem Hohlraum handeln.

In einer weiteren bevorzugten Ausführungsform handelt es sich bei dem Mittel (b) um ein Unterdruck-Anschlussstück (Port), welches auf einer der Innen- oder Außenseiten des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden. Es ist auch denkbar, dass der Port selbst über entsprechende Befestigungseinrichtungen, wie beispielsweise Klebeflächen, verfügt. Geeignete Unterdruck-Anschlussstücke sind kommerziell erhältlich. Dabei handelt es sich typischerweise um Unterdruck-Anschlussstücke, welche auf der Außenseite des Abdeckmaterials befestigt werden. Auch das Unterdruck-Anschlussstück verfügt zweckmäßigerweise über einen Unterdruckadapter, um mit den weiteren Komponenten des Unterdrucksystems verbindbar zu sein.

Neben den vorstehend beschriebenen Bestandteilen (a) und optional (b) umfasst die erfindungsgemäße Vorrichtung noch Bestandteil (c). Die Wundauflage (c), welche in der erfindungsgemäßen Vorrichtung Verwendung findet, wird nachstehend genauer beschrieben. Alle Erläuterungen zur Wundauflage (c), inklusive des Vliesstoffs (c1) und der Salbengrundlage (c2), betreffen nicht nur die erfindungsgemäße Vorrichtung, sondern auch das erfindungsgemäße Verfahren zur Herstellung der Wundauflage und die erfindungsgemäße Verwendung der Wundauflage in der Unterdrucktherapie.

Die Wundauflage (c) enthält einen Vliesstoff (c1) und eine Salbengrundlage (c2).

Üblicherweise enthält ein Vlies zusammen liegende Fasern, welche aber noch nicht mit einander verbunden sind. Aus diesem Grund beruht die Festigkeit eines Vlieses bevorzugt nur auf der fasereigenen Haftung. Die erwähnte Festigkeit kann dann durch eine geeignete Aufarbeitungsmethoden beeinflusst werden, sodass eine Verfestigung eintritt.

Unter einem Vliesstoff (c1) soll im Rahmen dieser Anmeldung ein Flächen- oder Raumgebilde aus gerichtet angeordneten oder wahllos zueinander befindlichen Fasern verstanden werden, welche mechanisch und/oder thermisch und/oder chemisch verfestigt wurden.

Vliesstoffe (im Englischem auch "nonwoven") sind wesentlich verschieden von Gewebe, Gestricken und Gewirken, welche sich durch eine vom Herstellverfahren bestimmte Legung der einzelnen Fasern oder Fäden auszeichnen.

Die Vliesstoffe können sich in der Faserart und deren Ursprung, dem Spinnverfahren zur Fasergewinnung und dem Verfestigungsverfahren unterscheiden.

Bei der Herstellung von Vliesstoffen können Fasern natürlichen oder synthetischen Ursprungs oder Gemische davon verwendet werden.

Zu den Fasern natürlichen Ursprungs zählen z.B. Seide, Cellulose, Baumwolle und Wolle.

Die Fasern synthetischen Ursprungs umfassen die synthetischen Polymere (Kunstfasern). Beispiele hier sind Viskose, Polyacrylate, Polyamide, Polyimide, Polyamidimide, Polyurethane, Polyester (insbesondere Polyethylenterephthalat und Polybutylenterephthalat), Polyetherestern, Polyethern Polyacrylnitril, Polyalkene (insbesondere Polyethylen und Polypropylen) Polyurethane und Polytetrafluorethylen. Bevorzugt sind Polyester.

Die Vliesstoffe können durch die Ausrichtung ihrer Fasern unterschieden werden. Es gibt Vliesstoffe, bei denen die Fasern eine Vorzugsrichtung aufweisen (faserorientierte Vliesstoffe) und Vliesstoffe, bei denen keine Vorzugsrichtung der Fasern vorliegt (Wirrfaservliesstoffe). In einer bevorzugten Ausführungsform werden Wirrfaservliesstoffe eingesetzt.

Die Spinnverfahren, die zur Fasergewinnung dienen können, sind beispielsweise das Trockenspinnverfahren, das Nassspinnverfahren, das Schmelzspinnverfahren und das Matrixspinnverfahren.

Die zur Verfestigungsverfahren, welche benötigt werden, um aus dem Vlies den eigentlichen Vliesstoff zu fertigen, können wie oben erwähnt mechanische und/oder thermische und/oder chemische Verfestigungsverfahren sein.

Mechanisch können Vliesstoffe durch Verfestigung des Vlieses/der Fasern mittels Vernadelung gewonnen werden. Hierbei bohren sich mit kleinen Kerben versehene Nadeln durch das Vlies und reißen dabei Faserbündel mit, welche Verschlingungen bilden und zur Verfestigung führen. Hierzu können unterschiedliche Typen von Nadeln, z.B. Kronennadeln eingesetzt werden. Anstelle der traditionellen Vernadelung wird heutzutage häufig eine Wasserstrahlvernadelung verwendet.

Die chemische Verfestigung des Vlieses zum Vliesstoff wird gewöhnlich durch die Zugabe von Bindemitteln, beispielsweise Elastomeren oder Plastomeren, erreicht. Chemische Verfestigungsverfahren umfassen dabei z.B. die Imprägnierung mit flüssigen oder schaumförmigen Bindemittel(n), das Aufdrucken von Bindemittel oder die Sprühverfestigung.

Unter thermischer Verfestigung wird das Verkleben von thermoplastischen Fasern miteinander oder von andern Fasern mit Hilfe von Thermoplasten verstanden. Hierzu können thermoplastische Bindefasern bereits bei der Faserherstellung hinzugemischt werden. Die thermische Aktivierung kann mittels Heißluft oder Kalander erfolgen. Seit einiger Zeit kann der Vliesstoff aber auch durch partielle Verschweißung durch Ultraschall hergestellt werden.

Die Verfestigungsverfahren können allein oder in Kombination untereinander durchgeführt werden. So kann beispielsweise ein Vlies durch leichte Vernadelung von der einen und thermische Verfestigung von der anderen Seite behandelt werden, um einen Vliesstoff zu erhalten.

In einer bevorzugten Ausführungsform liegen in mindestens 60% des faserartigen Bestandteils des Vliesstoffs (c1) Fasern vor, die ein Verhältnis von Länge zu Durchmesser von mindestens 350, bevorzugt 400 bis 650 aufweisen. Es hat sich gezeigt, dass durch das genannte Verhältnis eine vorteilhafte Steifigkeit des in der Anmeldung verwendeten Vliesstoffs erreicht wird. Bei einem größeren Verhältnis ist der Vliesstoff zu steif, während er bei einem kleineren Verhältnis zu nachgiebig ist.

In einer weiteren bevorzugten Ausführungsform haben die Fasern des Vliesstoffs einen Durchmesser von 0,1 bis 100 µm, bevorzugt 0,25 bis 75 µm, mehr bevorzugt 0,5 bis 50 µm, besonders bevorzugt 0,75 bis 25 µm, insbesondere 1 bis 10 µm.

In einer weiteren bevorzugten Ausführungsform weist der Vliesstoff eine Flächenmasse von 25 bis 850 g/m² auf, bevorzugt 50 bis 750 g/m², mehr bevorzugt 100 bis 650 g/m², insbesondere 200 bis 600 g/m² , gemessen nach DIN EN 29073-1 (Prüffläche 100 cm² (z.B. 10 cm x 10 cm), Angleichung auf das Normklima (23°C, 50% rF) bis zur Gewichtskonstanz, Bestimmung des Gewichts) auf.

Es hat herausgestellt, dass Vliesstoffe mit Fasern des obengenanten Durchmessers und der vorstehend genannten Flächenmasse von den Patienten als sehr angenehm in Bezug auf den Tragekomfort angesehen werden.

In einer weiteren bevorzugten Ausführungsform weist der Vliesstoff (c1) eine Dicke von 1 bis 75 mm, bevorzugt 3 bis 50 mm, mehr bevorzugt 5 bis 40 mm, insbesondere von 7 bis 35 mm, auf.

Es hat sich ferner gezeigt, dass vorstehend genannte Aufgaben unerwartet vorteilhaft gelöst werden können, wenn der Vliesstoff (c1) eine spezielle Luftdurchlässigkeit aufweist. In einer bevorzugten Ausführungsform weist der Vliesstoff (c1) eine Luftdurchlässigkeit von 200 bis 5000 l/(m²sec), mehr bevorzugt von 300 bis 3500 l/(m²sec), noch mehr bevorzugt von 350 bis 3000 l/(m²sec), insbesondere von 400 bis 2500 l/(m²sec), gemessen gemäß DIN EN ISO 9237 (20 mm Prüfdicke, 20 cm² Prüffläche, 100 Pa Differenzdruck), auf.

Grundsätzlich kann der Vliesstoff (c1) aus jedem beliebigen der oben genannten Materialien bestehen. Er sollte jedoch bestimmten physikalischen Anforderungen genügen. Es hat sich nämlich gezeigt, dass die vorstehend genannten Aufgaben unerwartet vorteilhaft gelöst werden können, wenn der Vliesstoff (c1) eine spezielle Zugfestigkeit, insbesondere in Produktionsrichtung und eine spezielle Dehnung, insbesondere entgegen der Produktionsrichtung aufweist. In einer bevorzugten Ausführungsform weist der Vliesstoff (c1) eine Zugfestigkeit in Produktionsrichtung von 25 N pro 5 cm bis 1000 N pro 5 cm, mehr bevorzugt 100 N pro 5 cm bis 900 N pro 5 cm, noch mehr bevorzugt 200 N pro 5 cm bis 800 N pro 5 cm, gemessen gemäß DIN EN 12127 auf. Ferner weist der Vliesstoff (c1) bevorzugt eine Dehnung, insbesondere entgegen der Produktionsrichtung von 25 % bis 750 %, mehr bevorzugt von 100 % bis 700 %, noch mehr bevorzugt von 200 % bis 600 %, gemessen gemäß DIN EN 29073 T3 auf.

Es hat sich weiterhin gezeigt, dass vorstehend genannte Aufgaben unerwartet vorteilhaft gelöst werden können, wenn der Vliesstoff (c1) eine Dichte zwischen 0,001 und 0,21 g/cm³ aufweist, mehr bevorzugt zwischen 0,005 und 0,15 g/cm³, noch mehr bevorzugt zwischen 0,01 und 0,10 g/cm³, insbesondere zwischen 0,012 und 0,05 g/cm³, aufweist, gemessen gemäß DIN 53885 (Prüfmuster von ausreichender Größe (bevorzugt 50 mm x 50 mm), Angleichung auf Normklima (23°C/ 50% rF) für mindestens 16 Stunden, Bestimmung von Dicke (bevorzugt mit Wolf Universal Dickenmessgerät DM 100) und Gewicht).

Es hat sich weiterhin gezeigt, dass vorstehend genannte Aufgaben unerwartet vorteilhaft gelöst werden können, wenn der Vliesstoff (c1) Silber in Form von Silberionen oder in Form von atomarem Silber enthält. Bevorzugt wird nach der Herstellung des Vliesstoffs (c1) eine Silberbeschichtung aufgebracht. Alternativ kann das Silber bereits auf das Vlies und damit vor der Verfestigung zum Vliesstoff aufgebracht werden. Bevorzugt enthält der Vliesstoff (c1) 0,000001 bis 0,1 Gew.-%, mehr bevorzugt 0,0001 bis 0,01 Gew.-% Silber, bezogen auf das Gesamtgewicht des Vliesstoffs (c1).

In einer bevorzugten Ausführungsform wird der Vliesstoff (c1) in trockenem Zustand mit der Salbengrundlage (c2) benetzt. Bevorzugt ist somit der Vliesstoff z.B. nicht mit einer Aktivierungslösung (wie z.B. Ringerlösung) getränkt.

Grundsätzlich sind als Salbengrundlage (c2) die im Fachgebiet bekannten und in Bauer, Frömming, Führer "Lehrbuch der Pharmazeutischen Technologie", 8. Auflage, Kapitel 12.1 bis 12.6 beschriebenen Salbengrundlagen geeignet. Demgemäß versteht man unter Salbengrundlagen streichfähige, halbfeste Zubereitungen, die grundsätzlich zur Anwendung auf der Haut oder auf den Schleimhäuten geeignet sind, jedoch (noch) keine pharmazeutischen Wirkstoffe enthalten. Die Herstellung von Salbengrundlagen ist dem Fachmann bekannt, es wird auf Ph. Eur. 6.0 "Halbfeste Zubereitungen zur kutanen Anwendung" verwiesen.

Bevorzugt werden Salbengrundlagen (c2) verwendet, die keine wässrige Phase enthalten. Bevorzugt werden hydrophobe, wasseraufnehmende und/oder hydrophile Salbengrundlagen verwendet. Hydrophobe Salbengrundlagen sind bevorzugt.

Hydrophobe Salbengrundlagen sind solche, die im Wesentlichen keine polaren Bestandteile enthalten und deshalb Wasser im Wesentlichen nicht aktiv binden. Es handelt sich daher um eine lipophile Grundlage, in die Wasser nur durch eine mechanische Dispergierung eingearbeitet werden kann. Beispiele für bevorzugte hydrophobe Salbengrundlagen sind Kohlenwasserstoffgrundlagen (z.B. Vaselin oder Vaselin-Paraffin-Mischungen), Diglyceride, Triglyceride, Wachse, Polyalkylsiloxane und Gemische daraus. Besonders bevorzugt werden Triglyceride oder Gemische aus Triglyceriden und Diglyceriden als Salbengrundlage (c2) verwendet.

Wasseraufnehmende Salbengrundlagen enthalten lipophile Stoffe und Tenside. Beispiele für wasseraufnehmende Salbengrundlagen sind W/O-Emulsionen (z.B. Wollwachs) oder O/W-Emulsionen.

Hydrophile Salbengrundlagen sind Zubereitungen, die mit Wasser mischbar sind. Bevorzugtes Beispiel ist eine Polyethylenglykol-Salbengrundlage (PEG mit gewichtsmittlerem Molekulargewicht von üblicherweise 300 bis 4000 g/mol, bevorzugt 1500 bis 3000 g/mol).

Neben den vorstehend beschriebenen bevorzugten Salbengrundlagen umfasst die Komponente (c2) auch noch Cremegrundlagen (hydrophile und hydrophobe Cremegrundlagen), Gele (hydrophobe Gele, hydrophile Gele) und Pasten (Suspensions-Salbengrundlagen).

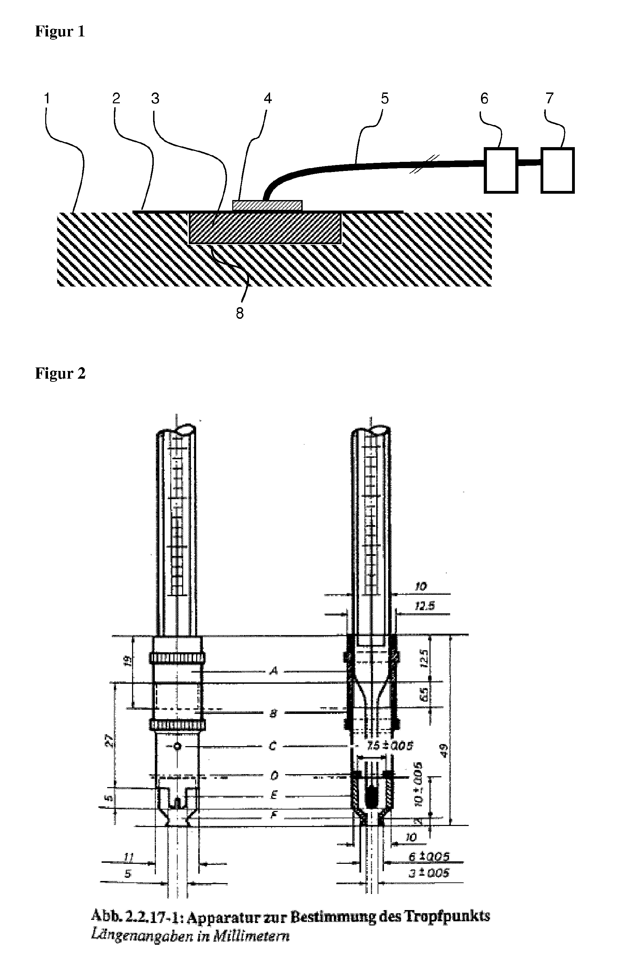

Die Salbengrundlage (c2) sollte von halbfester Konsistenz sein. Es hat sich zur Lösung der eingangs gestellten Aufgaben als vorteilhaft erwiesen, wenn die Salbengrundlage einen Tropfpunkt von 20 bis 80°C, bevorzugt von 25 bis 55°C, mehr bevorzugt von 30 bis 50°C, noch mehr bevorzugt von 33 bis 48°C und insbesondere von 35 bis 45°C, aufweist. Unter Tropfpunkt wird hierbei die Temperatur verstanden, bei welcher sich der erste Tropfen einer schmelzenden Substanz vom Metallnippel eines Tropfpunktthermometers löst. Zur experimentellen Bestimmung des Tropfpunkts wird auf die nachstehenden Erläuterungen zu Figur 2 verwiesen.

Neben dem Tropfpunkt hat es sich zur Lösung der eingangs beschriebenen Aufgaben als vorteilhaft erwiesen, wenn die Salbengrundlage (c2) einen oder mehrere der folgenden Parameter erfüllt:
Säurezahl von 0,001 bis 2,0 mg KOH/g, bestimmt gemäß Ph.Eur. 6.0, 2.5.1;
Iodzahl von 0,001 bis 3,0 g I2/100g, bestimmt gemäß Ph.Eur. 6.0, 2.5.4;
Peroxidzahl von 0,001 bis 1,0 mequi O/kg, bestimmt gemäß Ph.Eur. 6.0, 2.5.5 A; OH-Zahl von 1 bis 100, bevorzugt 5 bis 90 mg KOH/g, bestimmt gemäß Ph.Eur. 6.0, 2.5.3;
Verseifungszahl von 200 bis 350 mg KOH/g, bevorzugt von 240 bis 300 mg KOH/g, bestimmt gemäß Ph.Eur 6.0, 2.5.6;
Schwermetallgehalt von maximal 10 ppm, bestimmt gemäß Ph.Eur. 6.0, 2.4.8.D.

Sofern in den in dieser Anmeldung zitierten Normen und Ph.Eur.-Vorschriften nichts anderes angegeben ist, werden im allgemeinen die Testmethoden bei Normklima, d.h. bei 23°C und 50 % relativer Luftfeuchte, sowie bei einem Luftdruck von 1013 mbar durchgeführt.

In einer besonders bevorzugten Ausführungsform werden Triglyceride als Salbengrundlage (c2) verwendet. 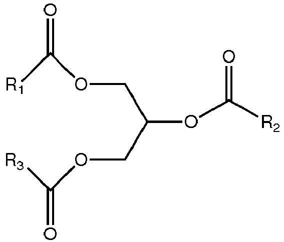 R₁, R₂ und R₃ können hierbei gleich oder bevorzugt unterschiedlich sein.

In einer bevorzugten Ausführungsform enthält das Triglycerid einen Glycerinrest und drei C₆-C₂₈-Säurereste, bevorzugt C₈-C₁₈-Säurereste. Die Säurereste können gesättigt oder ungesättigt sein, bevorzugt sind gesättigte Fettsäuren. Die Säurereste können gegebenenfalls substituiert sein, z.B. mit einer Hydroxygruppe.

Besonders bevorzugt enthalten die Säurereste der Triglyceride Caprylsäure, Caprinsäure, Laurinsäure und/oder Stearinsäure. Hierbei sind insbesondere Triglyceride bevorzugt, wobei die Säure Fettfraktion 20 bis 40 Gew.-% Caprylsäure, 10 bis 30 Gew.-% Caprinsäure, 5 bis 20 Gew.-% Laurinsäure und 30 bis 50 Gew.-% Stearinsäure enthält, insbesondere daraus besteht.

In einer alternativen, besonders bevorzugten Ausführungsform werden Diglyceride als Salbengrundlage (c2) verwendet. 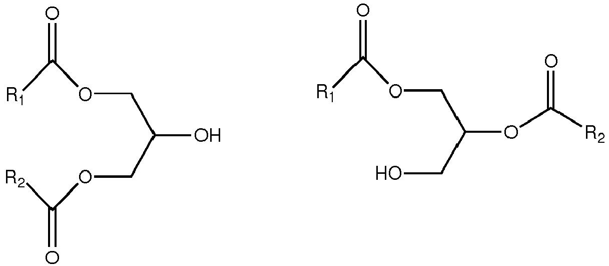 R₁ und R₂ können hierbei gleich oder bevorzugt unterschiedlich sein.

In einer bevorzugten Ausführungsform enthält das Diglycerid einen Glycerinrest und zwei C₄-C₂₈-Säurereste, bevorzugt C₆-C₁₈-Säurereste. Die Säurereste können gesättigt oder ungesättigt sein, bevorzugt sind gesättigte Fettsäuren. Die Säurereste können gegebenenfalls substituiert sein, z.B. mit einer Hydroxygruppe. Besonders bevorzugt enthalten die Säurereste der Isostearinsäure Stearinsäure, 12-Hydroxystearinsäure und/oder Adipinsäure.

In einer besonders bevorzugten Ausführungsform enthält die Salbengrundlage (c2) ein Gemisch aus Triglyceriden und Diglyceriden. Hierbei enthält die Salbengrundlage (c2) insbesondere
25 bis 90 Gew.-%, bevorzugt 45 bis 80 Gew.-% Triglyceride, und
10 bis 75 Gew.-%, bevorzugt 20 bis 55 Gew.-% Diglyceride.

Ebenfalls ist es bevorzugt, dass der Mischung aus Tri- und Diglyceriden noch Polyethylenglykol (PEG) zugesetzt wird. Insbesondere enthält die Mischung Tri-/DiGlyceride/PEG 1 bis 30 Gew.-%, bevorzugt 5 bis 20 Gew.-% Polyethylenglykol, bezogen auf das Gesamtgewicht der Mischung. Bevorzugt wird hierbei PEG mit einem gewichtsmittlerem Molekulargewicht von 500 bis 3000 g/mol, insbesondere von 1500 bis 2500 g/mol, verwendet.

Eine besonders bevorzugte Salbengrundlage (c2) enthält:
20 bis 90 Gew.-%, bevorzugt 55 bis 80 Gew.-% Triglyceride, insbesondere enthaltend Fettsäurereste, ausgewählt aus Caprylsäure, Caprinsäure, Laurinsäure und/oder Stearinsäure;
5 bis 75 Gew.-%, bevorzugt 15 bis 45 Gew.-% Diglyceride, insbesondere enthaltend Fettsäurereste, ausgewählt aus Isostearinsäure, Stearinsäure, 12-Hydroxystearinsäure und/oder Adipinsäure; und
0 bis 30 Gew.-%, bevorzugt 5 bis 20 Gew.-% Polyethylenglykol mit einem gewichtsmittleren Molekulargewicht von 500 bis 3000 g/mol.

Neben Di- und Triglyceriden kann die Salbengrundlage (c2) bevorzugt auch ferner Fettalkohole, alkoxylierte Fettalkohole, Fettsäuren und alkoxylierte Fettsäuren enthalten.

Die Salbengrundlage (c2) enthält bevorzugt keine Monoglyceride.

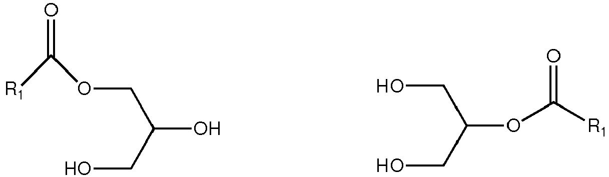

Insbesondere enthält die Salbengrundlage (c2) kein Glycerolmonooleat.

In einer bevorzugten Ausführungsform kann die Salbengrundlage (c2) auch Substanzen mit antimikrobieller Wirkung enthalten. Bei der Substanz mit antimikrobieller Wirkung kann es sich beispielsweise um Substanzen mit Amino- oder Iminogruppen handeln. Weiterhin kann es sich bei der Substanz mit antimikrobieller Wirkung um antimikrobiell wirksame Metallkationen handeln, insbesondere um Silberkationen, beispielsweise um einen Komplex von 1-Vinyl-2-pyrrolidon mit Silberkationen. Besonders geeignete Substanzen mit antimikrobieller Wirkung sind weiterhin Biguanid-Derivate wie Chlorhexidin oder Polybiguanide, wie Polyethylenbiguanid (PEB), Polytetramethylenbiguanid (PTMB) oder Polyethylenhexamethylenbiguanid (PEHMB). Ein besonders bevorzugtes Polybiguanid ist Polyhexamethylenbiguanid (PHMB bzw. Polyhexanid). Weitere geeignete Substanzen mit antimikrobieller Wirkung sind Polyguanidine wie zum Beispiel Polyhexamethylenguanidine (PHMG), N-Octyl-1-[10-(4-Octyliminopyridin-1-yl)decyl]pyridin-4-imin (Octenidin), quartäre Ammoniumverbindungen wie zum Beispiel Benzalkoniumchlorid oder Cetylpyridiniumchlorid, Triazine wie zum Beispiel 1-(3-Chloroallyl)-3,5,7-triaza-1-azonia-adamantan-Chlorid oder die Ammoniumverbindung Taurolidin.

Substanzen mit antimikrobieller Wirkung sind üblicherweise in der Salbengrundlage (c2) in einer Menge von 0 bis 15 Gew.-%, bevorzugt von 0,1 bis 5 Gew.-%, bezogen auf das Gesamtgewicht der Salbengrundlage (c2), enthalten.

Der Vliesstoff (c1) wird bevorzugt mit der Salbengrundlage (c2) benetzt. Unter "benetzen" wird hierbei verstanden, dass die Salbengrundlage die Oberflächen des Vliesstoffs bedeckt, bevorzugt vollständig bedeckt. In einer bevorzugten Ausführungsform werden mindestens 20 %, mehr bevorzugt mindestens 50 %, noch mehr bevorzugt mindestens 70 %, insbesondere mindestens 90 % der Gesamtoberfläche des Vliesstoffs (c1) mit Salbengrundlage (c2) bedeckt. Das Benetzen der Oberfläche des Vliesstoffs (c1) mit Salbengrundlage (c2) kann auch als Imprägnieren bezeichnet werden.

Es hat sich herausgestellt, dass durch eine spezielle Menge an Salbengrundlage (c2) die eingangs geschilderten Aufgaben besonders vorteilhaft gelöst werden können. In einer bevorzugten Ausführungsform beträgt der Anteil an Salbengrundlage (c2) 10 bis 95 Gew.-%, mehr bevorzugt 30 bis 85 Gew.-%, noch mehr bevorzugt 35 bis 80 Gew.-%, besonders bevorzugt 40 bis 75 Gew.-%, insbesondere 45 bis 70 Gew.-%, bezogen auf das Gesamtgewicht der Wundauflage (c). Das Gesamtgewicht der Wundauflage (c) ergibt sich aus der Addition der Gewichte der Bestandteile (c1) und (c2).

Grundsätzlich gilt, dass alle Erläuterungen zu bevorzugten Ausführungsformen einzelner Parameter des Vliesstoffs (c1) und/oder der Salbengrundlage (c2) nicht isoliert zu betrachten sind, sondern auch in Kombination mit den Erläuterungen zu bevorzugten Ausführungsformen anderer Parameter beziehungsweise in Kombination mit den Erläuterungen zu den Stoffzusammensetzungen von (c1) und (c2) gelten.

Die Wundauflage (c) enthaltend (c1) und (c2) kann vorteilhaft durch das erfindungsgemäße Verfahren hergestellt werden. Dieses umfasst die Schritte:
(I) Erwärmen einer Salbengrundlage, bevorzugt über den Tropfpunkt;
(II) Einbringen des Vliesstoffs in die erwärmte Salbengrundlage, so dass der Vliesstoff mit der Mischung aus Salbengrundlage benetzt wird;
(III) gegebenenfalls temporäres Zusammendrücken des Vliesstoffs, bevorzugt auf mindestens 90 % und höchstens 10 % seines ursprünglichen Volumens, um eine Benetzung der Oberfläche des Vliesstoffs mit der Salbengrundlage zu erreichen; und
(IV) gegebenenfalls Entfernen der überschüssigen Salbengrundlage, bevorzugt durch Ausdrücken des Vliesstoffs.

In Schritt (I) wird die Salbengrundlage erwärmt, bevorzugt auf etwa 10 bis 30°C über den Tropfpunkt.

In Schritt (II) wird der Vliesstoff in die erwärmte Salbengrundlage eingebracht und bevorzugt untergetaucht. Die Verweilzeit in der Mischung aus Schritt (II) ist abhängig von der Größe des Vliesstoffs und beträgt üblicherweise 10 bis 100 Sekunden.

Schritt (III) kann während oder nach, bevorzugt während dem Untertauchen erfolgen.

Unter temporäres Zusammendrücken des Vliesstoffs wird hier verstanden, dass der Vliesstoff manuell oder durch eine geeignete Maschine zusammengedrückt und danach wieder losgelassen wird. Dabei kann der Vliesstoff unmittelbar nach dem Zusammendrücken wieder losgelassen werden oder auch für kurze Zeit, beispielsweise für 1 bis 10 Sekunden, im zusammengedrückten Zustand gehalten und dann losgelassen werden. Nach dem auf das temporäre Zusammendrücken folgende Loslassen des Vliesstoffs kann der Vliesstoff dann wieder vollständig oder weitgehend sein ursprüngliches Volumen einnehmen. Während des Relaxierungsvorgangs wird üblicherweise die Salbengrundlage aufgenommen, wobei bevorzugt eine vollständige Benetzung der Oberfläche des Vliesstoffs mit der Salbengrundlage erreicht wird.

Anschließend wird der mit Salbengrundlage benetzte Vliesstoff entnommen. Überschüssige Salbengrundlage kann in Schritt (IV) ausgedrückt werden. Bevorzugt erfolgt das Ausdrücken, bevor die Salbengrundlage unter den Tropfpunkt abgekühlt ist. Bevorzugt wird die Ausdrückkraft so gewählt, dass der Anteil an Salbengrundlage 10 bis 95 Gew.-% beträgt, mehr bevorzugt 30 bis 85 Gew.-%, noch mehr bevorzugt 35 bis 80 Gew.-%, besonders bevorzugt 40 bis 75 Gew.-%, insbesondere 45 bis 70 Gew.-%, bezogen auf das Gesamtgewicht der Wundauflage.

Alle vorstehend aufgeführten Erläuterungen zu bevorzugten Ausführungsformen der Komponenten (c), (c1) und (c2) finden auch für das erfindungsgemäße Verfahren Anwendung.

Eine bevorzugte Ausführungsform ist eine Wundauflage, die durch das erfindungsgemäße Verfahren erhalten wird.

Weiterhin stellt die Erfindung ein gebrauchsfertiges Set zur Unterdruck-Wundbehandlung, umfassend die erfindungsgemäße Vorrichtung, bereit, wobei der erfindungsgemäße Vliesstoff (c1) als Wundauflage (c) geeignet ist und gebrauchsfertig abgepackt vorliegt.

Gegenstand der Erfindung ist somit ein gebrauchsfertiges Set zur Unterdruck-Wundbehandlung, umfassend
(a) Abdeckmaterial zum luftdichten Verschließen eines Wundraums, d.h. der Wunde und der Wundumgebung;
(b) gegebenenfalls ein Mittel zum Anschließen einer Unterdruckquelle, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind,
   und
(c) eine gebrauchsfertig abgepackte Wundauflage, enthaltend
(c1) einen Vliesstoff, der mit
(c2) einer Salbengrundlage benetzt ist, die bevorzugt einen Tropfpunkt von 20 bis 80°C aufweist. Bevorzugt beträgt der Anteil an Salbengrundlage (c2) 10 bis 95 Gew.-% beträgt, bezogen auf das Gesamtgewicht der Wundauflage. Bevorzugt handelt es sich um eine Triglycerid-Salbengrundlage.

Die vom Set umfasste abgepackte Wundauflage (c) wird bevorzugt feuchtigkeitsdicht abgepackt. Vorzugsweise liegt die gebrauchsfertige Wundauflage in steriler Form vor, wobei die Sterilisation durch Ethylenoxidgas-Behandlung und/oder durch Bestrahlung erfolgen kann. Die Sterilisation durch Bestrahlung ist bevorzugt. Die Bestrahlungssterilisation kann mit Beta- und/oder Gammastrahlen durchgeführt werden. Beta-Strahlung ist bevorzugt. Die Sterilisation kann durch Bestrahlung mit einer Dosis von 20 bis 60 kGy (20 bis 60 kJ/kg) erreicht werden. Es hat sich herausgestellt, dass bevorzugt eine Dosis von 25 bis 40 kGy (25 bis 40 kJ/kg) eingesetzt wird. Alternativ ist auch Gamma-Strahlung bevorzugt.

Das Set kann weitere optionale Bestandteile wie beispielsweise Klebemittel zum Fixieren des Verbands, Abdichtmittel zur Herstellung einer luftundurchlässigen Abdichtung des Verbands, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Bevorzugt enthält das erfindungsgemäße Set ferner Schere, Tupfer und/oder Pinzette, insbesondere in steriler Form.

Das Set kann sowohl die mindestens eine Wundkontaktschicht als auch mindestens eine zusätzliche Druckverteilungsschicht umfassen. Vorzugsweise umfasst das Set weiterhin eine gebrauchsfertige Unterdruckeinheit.

Ein weiterer Gegenstand der Erfindung ist die Verwendung der vorstehend erläuterten Wundauflage (c) zur oder in der Unterdrucktherapie von Wunden, d.h. die Verwendung eines Vliesstoffs (c1), der mit einer Salbengrundlage (c2) benetzt ist, zur Unterdrucktherapie von Wunden, insbesondere als Wundauflage (c). Ein weiterer bevorzugter Gegenstand der Erfindung ist eine Wundauflage (c), die einen Vliesstoff (c1) und eine Salbengrundlage (c2) enthält, zur Unterdrucktherapie von Wunden. Ein weiterer bevorzugter Gegenstand der Erfindung ist ein Verfahren zur Behandlung einer Wunde mit einer Wundauflage gemäß der vorliegenden Anmeldung.

Alle vorstehend aufgeführten Erläuterungen zu bevorzugten Ausführungsformen der Komponenten (c), (c1) und/oder (c2) finden sowohl einzeln als auch in Kombination auch für die erfindungsgemäße Verwendung Anwendung.

So betrifft beispielsweise die Erfindung die Verwendung eines Vliesstoff (c1),
wobei der Vliesstoff bevorzugt eine Dehnung, insbesondere entgegen der Produktionsrichtung von 25 % bis 750 % aufweist, mehr bevorzugt von 100 % bis 700 %, noch mehr bevorzugt von 200 % bis 600 %, gemessen gemäß DIN EN 29073-1, wobei der Vliesstoff bevorzugt eine Zugfestigkeit insbesondere in Produktionsrichtung von 25 N pro 5 cm bis 1000 N pro 5 cm aufweist, mehr bevorzugt 100 N pro 5 cm bis 900 N pro 5 cm, noch mehr bevorzugt 200 N pro 5 cm bis 800 N pro 5 cm, gemessen gemäß DIN EN 12127;
wobei der Vliesstoff bevorzugt eine Dichte zwischen 0,001 und 0,21 g/cm³ aufweist, mehr bevorzugt wischen 0,005 und 0,15 g/cm³, noch mehr bevorzugt zwischen 0,01 und 0,10 g/cm³, insbesondere zwischen 0,012 und 0,05 g/cm³, aufweist, gemessen gemäß DIN 53885,
wobei der Vliesstoff bevorzugt eine Luftdurchlässigkeit von 200 bis 5000 l/(m²sec) aufweist, mehr bevorzugt von 300 bis 3500 l/(m²sec), noch mehr bevorzugt von 350 bis 3000 l/(m²sec), besonders bevorzugt von 400 bis 2500 l/(m²sec), insbesondere von 500 bis 1000 l/(m²sec), gemessen gemäß DIN EN ISO 9237 (20 mm Prüfdicke, 20 cm² Prüffläche, 100 Pa Differenzdruck); und
wobei der Vliesstoff (c1) eine Flächenmasse von 25 bis 850 g/m² aufweist, bevorzugt 50 bis 750 g/m², mehr bevorzugt 100 bis 650 g/m², insbesondere 200 bis 600 g/m², gemessen gemäß DIN EN 29073-1.

In der erfindungsgemäßen Verwendung ist dieser Vliesstoff (c1) mit einer Salbengrundlage (c2) benetzt, wobei die Salbengrundlage (c2)
einen Tropfpunkt von 20 bis 80°C, bevorzugt von 25 bis 55°C, mehr bevorzugt von 30 bis 50°C, noch mehr bevorzugt von 33 bis 48°C und insbesondere von 35 bis 45°C aufweist;
bevorzugt aus einer hydrophoben, Wasser aufnehmenden und/oder hydrophilen Salbengrundlage ausgewählt ist;
bevorzugt Triglyceride enthält und insbesondere ein Gemisch aus Triglyceriden und Diglyceriden, z.B. 25 bis 90 Gew.-%, bevorzugt 45 bis 80 Gew.-% Triglyceride, und 10 bis 75 Gew.-%, bevorzugt 20 bis 55 Gew.-% Diglyceride, enthält.

Besondere Vorteile durch die erfindungsgemäße Vorrichtung, durch das erfindungsgemäße Set oder die erfindungsgemäße Verwendung, beziehungsweise Anwendung, ergeben sich, wenn es sich bei den Wunden um Brandwunden, um durch mechanische Traumatisierung entstandene Wunden, um eine durch Einwirkung von Chemikalien entstandene chronische Wunde, um eine durch eine Stoffwechselstörung verursachte chronische Wunde, um eine durch eine Durchblutungsstörungen verursachte chronische Wunde, oder um eine durch ein Druckgeschwür verursachte Wunde handelt.

In einer weiteren bevorzugten Ausführungsform wird die Wundauflage (c) (enthaltend (c1) und (c2) zur Anwendung in der Unterdrucktherapie bei der Behandlung einer durch eine Hauttransplantation entstandenen Wunde bereit gestellt. Die Anwendung umfasst die Behandlung von durch Spalthaut-Transplantationen und von durch Vollhaut-Transplantationen entstandenen Wunden mittels Unterdrucktherapie. Vorteilhafte Wirkungen ergeben sich durch die Struktur des Vliesstoffs (c1), der mit einer Salbengrundlage (c2) benetzt ist, sowie durch die gleichmäßige Druckverteilung. Bei der Anwendung der Wundauflage (c) bei der Behandlung einer durch eine Hauttransplantation entstandenen Wunde kann das Transplantat ("Skin-Graft") ausreichend fixiert und gleichzeitig können schädliche Scherkräfte vermieden werden.

Die vorstehend beschriebene Wundauflage (c) kann in der Unterdrucktherapie von Druckwunden bei Patienten mit einem Body-Mass-Index (BMI = Körpermasse durch Körperlänge im Quadrat) von weniger als 18,0, insbesondere mit einem Body-Mass-Index von 14 bis 17,5 vorteilhaft verwendet werden. Dies gilt insbesondere, wenn es sich um Patienten mit einem Alter von mehr als 60 Jahren handelt. Insbesondere bei diesen Patienten zeigt sich die vorteilhafte Wirkung der erfindungsgemäßen Vorrichtung oder des erfindungsgemäßen Sets.

Ein weiterer Gegenstand der Erfindung ist ein Verfahren zur Unterdrucktherapie von Wunden, umfassend die Schritte
a) Bereitstellen einer Vorrichtung nach einem der Ansprüche 1 bis 10;
b) Anlegen des Unterdruckverbandes an der Wunde;
c) Herstellung eines Unterdruckes von 20 mm Hg bis 250 mm Hg im Wundraum für mindestens 30 Minuten und höchstens 7 Tage.

Weitere bevorzugte Ausführungsformen der vorliegenden Erfindung werden durch die folgenden Punkte erläutert.
1. Vorrichtung zur Unterdrucktherapie von Wunden umfassend,
   (a) ein Abdeckmaterial zum luftdichten Verschließen eines Wundraums;
   (b) gegebenenfalls ein Mittel zum Anschließen einer Unterdruckquelle; und
   (c) eine Wundauflage, enthaltend
   (c1) einen Vliesstoff, der mit
   (c2) einer Salbengrundlage benetzt ist, wobei der Anteil an Salbengrundlage (c2) 10 bis 95 Gew.-% beträgt, bezogen auf das Gesamtgewicht der Wundauflage.
2. Vorrichtung nach Punkt 1, wobei die Salbengrundlage einen Tropfpunkt von 20 bis 80°C aufweist.
3. Vorrichtung nach Punkt 1 oder 2, wobei die Salbengrundlage (c2) ein Triglycerid enthält, das einen Glycerinrest und drei C₆-C₂₈-Säurereste, insbesondere C₈-C₁₈-Säurereste, enthält.
4. Vorrichtung nach einem der vorangegangen Punkte, wobei die Salbengrundlage (c2) ein Diglycerid enthält, das einen Glycerinrest und zwei C₄-C₂₈-Säurereste, insbesondere C₆-C₁₈-Säurereste, enthält.
5. Vorrichtung nach einem der vorhergehenden Punkte, wobei der Vliesstoff (c1) eine Dicke von 1 bis 75 mm aufweist.
6. Vorrichtung nach einem der vorhergehenden Punkte, wobei die einzelnen Fasern des Vliesstoffs (c1) eine Dicke von 0,25 bis 75 µm aufweisen.
7. Vorrichtung nach einem der vorangehenden Punkte, wobei der Vliesstoff (c1) eine Flächenmasse von 25 bis 850 g/m² aufweist.
8. Vorrichtung nach einem der vorangehenden Punkte, wobei der Vliesstoff (c1) eine Luftdurchlässigkeit von 200 bis 5000 l/(m²sec), gemessen gemäß DIN EN ISO 9237, aufweist.
9. Vorrichtung nach einem der vorangehenden Punkte, wobei der Vliesstoff (c1) eine Dichte zwischen 0,001 und 0,21 g/cm³ aufweist, gemessen gemäß DIN 53885.
10. Vorrichtung nach einem der vorangehenden Punkte, wobei das Abdeckmaterial (a) eine Wasserdampfdurchlässigkeit von 100 bis 2500 g/m² x 24 h aufweist, gemessen gemäß DIN EN 13726-2.
11. Verfahren zur Herstellung einer Wundauflage, umfassend die Schritte
   (I) Erwärmen einer Salbengrundlage, bevorzugt über den Tropfpunkt;
   (II) Einbringen des Vliesstoffs in die erwärmte Salbengrundlage, so dass der Vliesstoff mit der Salbengrundlage benetzt wird;
   (III) gegebenenfalls temporäres Zusammendrücken des Vliesstoffs, bevorzugt auf mindestens 90 % und höchstens 10 % seines ursprünglichen Volumens, um eine Benetzung der Oberfläche des Vliesstoffs mit der Salbengrundlage zu erreichen; und
   (IV) gegebenenfalls Entfernen der überschüssigen Salbengrundlage, bevorzugt durch Ausdrücken des Vliesstoffs.
12. Verfahren gemäß Punkt 11, wobei die Ausdrückkraft so gewählt wird, dass der Anteil an Salbengrundlage 10 bis 95 Gew.-% beträgt, bezogen auf das Gesamtgewicht der Wundauflage.
13. Wundauflage, erhältlich durch ein Verfahren gemäß Punkt 11 oder 12.
14. Vliesstoff, der mit einer Triglycerid-Salbengrundlage benetzt ist, zur Verwendung als Wundauflage in der Unterdrucktherapie von Wunden.
15. Wundauflage gemäß Punkt 14, wobei es sich um Brandwunden, um durch mechanische Traumatisierung entstandene Wunden, um durch Einwirkung von Chemikalien entstandene Wunden, um durch Stoffwechselstörungen verursachte Wunden, um durch Durchblutungsstörungen verursachte Wunden oder um durch Druckgeschwüre verursachte Wunden handelt.

### FIGUREN

- **Figur 1:**: Schematischer Aufbau der erfindungsgemäßen Vorrichtung (Seitenansicht)
- **Figur 2:**: Apparatur zu Bestimmung des Tropfpunkts

### Figurenlegende Figur 1:

- 1: Wundumgebung (d.h. in der Regel unversehrte Haut)
- 2: Luftundurchlässiges Abdeckmaterial (a)
- 3: Wundauflage (c) = Vliesstoff (c1), benetzt mit Salbengrundlage (c2)
- 4: Unterdruck-Anschlussstück (Port)
- 5: Unterdruck-Verbindungsleitung
- 6: Sammelgefäß
- 7: Unterdruckeinheit
- 8: Wundgrund

Die erfindungsgemäße Vorrichtung zur Unterdrucktherapie von Wunden wird anhand von **Figur 1** näher erläutert. Die Erfindung soll jedoch nicht auf die in den Zeichnungen oder in der Beschreibung der Zeichnungen dargestellten Ausgestaltungen reduziert verstanden werden. Vielmehr umfasst die erfindungsgemäße Vorrichtung auch Kombinationen der Einzelmerkmale der alternativen Formen.

In Figur 1 wird der schematische Aufbau der erfindungsgemäßen Vorrichtung in der Seitenansicht dargestellt. Die Vorrichtung umfasst ein luftundurchlässiges Abdeckmaterial (2), ein Mittel (4-5) zur funktionellen Verbindung des Wundraums mit einer außerhalb des Abdeckmaterials befindlichen Unterdruckquelle (7) sowie der Wundauflage (3), enthaltend Vliesstoff (c1) und Salbengrund (c2). Das Abdeckmaterial (2) ist im Bereich der Wundumgebung (1), welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung (1) befestigt werden kann. Das Abdeckmaterial (2) kann in verschiedenen Abmessungen und Formen vorliegen, beispielsweise kreisförmig, oval oder rechteckig. Es kann auch in einer an die Wunde angepassten, unregelmäßigen Form vorliegen. Das Abdeckmaterial (2) wird üblicherweise im Bereich der Wundumgebung (1) befestigt und luftdicht abgedichtet. Dies kann beispielsweise dadurch erfolgen, dass das Abdeckmaterial (2) einen Kleberand aufweist. Alternativ kann eine klebende Substanz entweder auf den Rand des Abdeckmaterials (2) und/oder auf die intakte Haut im Bereich der Wundumgebung aufgebracht werden. Dies hat den Vorteil, dass eine Anpassung des Abdeckmaterials an die Form und Größe der Wunde leichter möglich ist. Das Unterdruckanschlussstück (4) ist bei der hier gezeigten bevorzugten Ausführungsform auf der von der Wunde weg weisenden Außenseite des luftundurchlässigen Abdeckmaterials (2) angebracht. Um den Wundraum mit einer außerhalb des Abdeckmaterials befindlichen Unterdruckeinheit (7) funktionell zu verbinden, muss sich bei dieser Anordnung im Bereich des Unterdruckanschlussstücks (4) eine oder mehrere durch das Abdeckmaterial (2) hindurchgehende Öffnung(en) befinden.

Weiterhin ist eine luftdichte Abdichtung zu gewährleisten. Eine solche Abdichtung kann beispielsweise hergestellt werden, indem auf der von der Wunde weg weisenden Oberseite des Ports eine Folie (in Figur 1 nicht gezeigt) aufgebracht wird, die mit dem Abdeckmaterial (2) verklebt wird. Das Anlegen des Verbands kann erleichtert werden, wenn ein Port verwendet wird, bei dem ein geeignetes Befestigungs- und Abdichtmittel zum Befestigen des Ports auf dem Abdeckmaterial bereits vorhanden ist. Dies ist beispielsweise bei dem im Handel erhältlichen PPM-Drainageport^{®} der Firma Phametra-Pharma und Medica-Trading GmbH (Herne/Ruhrstadt, Deutschland) der Fall.

In einer bevorzugten Ausführungsform der Erfindung umfasst die Vorrichtung zur Unterdrucktherapie von Wunden keine Wundkontaktschicht zum Einbringen zwischen Wundauflage (3) und Wundoberfläche (8).

**Figur 2** zeigt die Apparatur zur Bestimmung des Tropfpunktes. Experimentell wird die Bestimmung wie folgt durchgeführt:

| | |
|---|---|
| Prüfgeräte: | - Tropfpunktthermometer nach Ubbelohde |
| | - 0 bis 110°C, geeicht |
| | - Becherglas, 1000 ml |
| | - Reagenzglas, ca. 200 mm lang, Durchmesser: ≈ 40 mm, mit durchbohrten Stopfen |
| | - Magnetrührer mit Heizplatte |
| | - Filterpapierzuschnitte 10 x 10 mm |
| Reagenzien: | - demineralisiertes Wasser |
| | - Apparatur zur Bestimmung des Tropfpunktes gemäß Figur 2. |

Die Apparatur (siehe Figur 2) besteht aus 2 zusammengeschraubten Metallhülsen (A) und (B). Die Hülse (A) ist an einem Quecksilberthermometer befestigt. Ein Metallnippel (F) ist lose am unteren Teil der Hülse (B) mit 2 Klemmbacken (E) befestigt. Sperrstifte (D) von 2 mm Länge fixieren genau die Lage des Nippels. Sie dienen ebenfalls dem Zentrieren des Thermometers. Eine Öffnung (C) in der Wand der Hülse (B) dient als Druckausgleich.

Die Abtropffläche des Nippels muss plan und die Ränder der Austrittsöffnung müssen im rechten Winkel dazu sein. Der untere Teil des Quecksilberthermometers hat die Form und die Dimension wie in der Abbildung angegeben. Das Thermometer erlaubt Temperaturmessungen von 0 bis 110°C, seine Skaleneinteilung beträgt 1°C (je 1 mm). Das Quecksilbergefäß des Thermometers hat einen Durchmesser von 3,5 ± 0,2 mm und eine Höhe von 6,0 ± 0,3 mm.

Die ganze Apparatur wurde in die Mitte eines etwa 200 mm langen Reagenzglases von 40 mm äußerem Durchmesser mit Hilfe eines durchbohrten Stopfens, durch welchen das Thermometer gesteckt wurde, eingehängt. Der Stopfen hat an der Seite eine Einkerbung. Die Nippelöffnung muss sich 15 mm über dem Boden des Reagenzglases befinden. Das Ganze wurde in ein mit Wasser gefülltes 1-Liter-Becherglas getaucht. Der Reagenzglasboden muss etwa 25 mm über dem Becherglasboden sein. Das Niveau des Wassers muss den oberen Teil der Hülse (A) erreichen. Ein Rührer sorgt für gleichmäßige Badtemperatur.

Das Prüfverfahren wurde wie folgt durchgeführt.

Wenn nichts anderes vorgeschrieben ist, wurde der Nippel vollständig mit der ungeschmolzenen zu prüfenden Substanz gefüllt. Mit einem Spatel wurde der Substanzüberschuss an beiden Enden des Nippels abgestrichen. Die Hülsen (A) und (B) wurden zusammengeschraubt und der Nippel bis zu den Sperrstiften in die Hülse (B) eingeschoben. Die durch das Thermometer ausgestoßene Substanz an der Nippelöffnung wurde mit einem Spatel abgestrichen. Die Apparatur wurde, wie oben beschrieben, in das Wasserbad gehängt. Anschließend wurde das Wasserbad so erwärmt, dass von etwa 10°C unterhalb des zu erwartenden Tropfpunktes die Temperatur um etwa 1 °C je Minute stieg. Die Temperatur wurde abgelesen, als der erste Tropfen vom Nippel abfiel. Die Bestimmung wurde mindestens dreimal mit neuen Proben durchgeführt, dabei durften die einzelnen Werte höchstens 3°C voneinander abweichen.

Auswertung: Als Tropfpunkt gilt der Mittelwert von drei Bestimmungen.

Die Erfindung soll durch die nachfolgenden Beispiele veranschaulicht werden.

### BEISPIELE

### Beispiel 1: Herstellung der erfindungsgemäßen Wundauflage (c)

Eine Salbengrundlage enthaltend eine Trigylcerid/Diglyceridmischung (Tropfpunkt ca. 40°C) wurde auf 55°C erwärmt. Ein Vliesstoff (Flächenmasse nach DIN EN 29073-1: 473 g/m²; Luftdurchlässigkeit nach EN ISO 9237: 556 l/(m²s); Dicke: 28 mm) wurde eingetaucht und etwas zusammengepresst. Nach dem Herausnehmen wurde der Vliesstoff ausgedrückt, so dass der Anteil an Salbengrundlage nach dem Ausdrücken 63 Gew.-% betrug.

### Beispiel 2: Test der Wundauflage gemäß Beispiel 1 im Wundsimulator

Die Wundauflage gemäß Beispiel 1 wurde im Unterdruck-Wundsimulator (beschrieben in DE 10 2008 064 510 A1) getestet.

Für eine "gut durchgeführte" Unterdrucktherapie-Simulation mussten die Ergebnisse des Tests lediglich sehr geringe Druckunterschiede zwischen dem Wundbereich (Sensor 1) und dem Portbereich (Sensor 2) sowie ein über einen langen Zeitraum gleichförmiges Verhalten aufweisen, bei gleichzeitiger linearer Ausführung bezüglich der entnommenen Exsudate. Der etwaige Druckunterschied ist auf den Wundverband zurückzuführen. Der Unterdruck wurde mittels eines ATMOS S041 Wunddrainagesaugers erzeugt. Das Exsudat wurde von einer B. Braun Perfusor^{®} F Spritzpumpe erzeugt, die einen konstanten Fluss erzeugen kann. Es wurde ein PPM Drainage Port-System (Firma Herne/Ruhrstadt, Deutschland) angewandt.

**Tabelle 1: Parameter**

| Wundverband | Temperatur (°C) | Druck (mmHg) | Exsudat | | Wundgröße (cm²) |
|---|---|---|---|---|---|
| | | | Fluss (ml.h⁻¹) | Viskosität (mPa•s) (Xanthan) | |
| erfindungsgemäße Wundauflage gemäß Beispiel 1 | 23 | 125 | 10 | 98 | 29,6 |

Die Xanthanlösung wird durch Mischen von 1000 g demineralisiertem Wasser, 2 g Xanthan und 0,2 g Allura Red hergestellt.

Die Wundauflage wurde auf den Wundsimulator aufgebracht und mit Hydrofilm^{®} bedeckt, um ein luftdichtes System zu erzeugen. Über der künstlichen Wunde wurde ein kleines Loch in den Film geschnitten und ein PPM-Port wurde angebracht, damit das Exsudat aus dem Wundbereich herausfließen kann. Dieser Port wurde zum einen mit einem Behälter verbunden, um das Exsudat zu sammeln und zum anderen mit dem Druckmesser verbunden, um den Druck innerhalb des Port-Systems zu messen. Der Behälter wurde mit einer Vakuumpumpe verbunden und die Menge des extrahierten Exsudats gemessen, indem das Gewicht des Exsudats mittels einer Skala bestimmt wurde. Der andere Drucksensor, der innerhalb des Wundsimulators angebracht wurde, maß den Druck innerhalb der simulierten Wunde. Der Versuch wurde gemäß den Parametern in Tabelle 1 durchgeführt.

### Datenanalyse

Der Versuch wurde über einen Zeitraum von 24 Stunden durchgeführt. Im Nachfolgenden werden die Ergebnisse angegeben, die für die beiden oben genannten Drücke (Figur 3) erhalten wurden. Sensor 1 maß den Druck innerhalb der Wunde, während Sensor 2 den Druck innerhalb des Port-Systems bestimmte. Hierbei wurden Mittelwerte für die jeweiligen Drücke und deren Druckunterschiede erhalten (Tabelle 2).

**Tabelle 2**

| Sensor 1 Mittelwert (mmHg): |
|---|
| 121 |
| |

| Sensor 2 Mittelwert (mmHg): |
|---|
| 123 |
| |

| Druckunterschied Mittelwert (mmHg): |
|---|
| **2** |

Die Ergebnisse zeigen, dass der Druckunterschied während des gesamten Versuchs gering und das Verhalten der Drücke normal blieb. Die errechneten und gesammelten Exsudate zeigten eine sehr ähnliche Kurve, was zudem den Erfolg des Versuchs bewies.

Daraus ergibt sich folgende Schlussfolgerung.

Die Menge des gesammelten Exsudats nahm gemäß den Erwartungen linear zu und der Druckunterschied zeigte sehr gute Ergebnisse. Dies bedeutet, dass die erfindungsgemäße Wundauflage gemäß Beispiel 1 in einem NPWT-Simulations-verfahren beständig und unerwartet vorteilhaft funktionierte.

## Patentansprüche

1. Vliesstoff, der mit einer Triglycerid-Salbengrundlage benetzt ist, zur Verwendung als Wundauflage in der Unterdrucktherapie von Wunden.

2. Vliesstoff zur Verwendung als Wundauflage gemäß Anspruch 1, wobei es sich bei den Wunden um Brandwunden, um durch mechanische Traumatisierung entstandene Wunden, um durch Einwirkung von Chemikalien entstandene Wunden, um durch Stoffwechselstörungen verursachte Wunden, um durch Durchblutungsstörungen verursachte Wunden oder um durch Druckgeschwüre verursachte Wunden handelt.

3. Vliesstoff zur Verwendung als Wundauflage gemäß Anspruch 1 oder 2 wobei die Fasern des Vliesstoffes eine Dicke von 0,25 bis 75 µm aufweisen.

4. Vliesstoff zur Verwendung als Wundauflage gemäß einem der vorstehenden Ansprüche, wobei die Flächenmasse des Vliesstoffs 25 bis 850 g/m² beträgt.

5. Vliesstoff zur Verwendung als Wundauflage gemäß einem der vorstehenden Ansprüche, wobei der Vliesstoff eine Luftdurchlässigkeit von 200 bis 5000l/(m²sec) gemessen gemäß DIN EN ISO 9237, aufweist.

6. Vliesstoff zur Verwendung als Wundauflage gemäß einem der vorstehenden Ansprüche, wobei der Vliesstoff eine Dicke von 1 bis 75 mm aufweist.

7. Vliesstoff zur Verwendung als Wundauflage gemäß einem der vorstehenden Ansprüche, wobei der Vliesstoff eine Dichte zwischen 0,001 und 0,21 g/cm³ aufweist, gemessen gemäß DIN 53885.

8. Vliesstoff zur Verwendung als Wundauflage gemäß einem der vorstehenden Ansprüche, wobei die Triglycerid-Salbengrundlage ein Triglycerid enthält, das einen Glycerinrest und drei C₆-C₂₈-Säurereste enthält.

9. Vliesstoff zur Verwendung als Wundauflage gemäß Anspruch 8, wobei die Triglycerid-Salbengrundlage ein Triglycerid enthält, das einen Glycerinrest und drei C₆-C₁₈-Säurereste enthält.

10. Vliesstoff zur Verwendung als Wundauflage gemäß Anspruch 8 oder 9,wobei die Triglycerid-Salbengrundlage ein Triglycerid enthält, wobei der Säurerest eine gesättigte Fettsäure ist.

11. Vliesstoff zur Verwendung als Wundauflage gemäß einem der Ansprüche 8 bis 10,wobei die Triglycerid-Salbengrundlage ein Triglycerid enthält, wobei der Säurerest ausgewählt ist aus Caprylsäure, Caprinsäure, Laurinsäure und/oder Stearinsäure.

12. Vliesstoff zur Verwendung als Wundauflage gemäß einem der vorstehenden Ansprüche, wobei die Triglycerid-Salbengrundlage einen Tropfpunkt von 20 bis 80°C aufweist.

13. Vliesstoff zur Verwendung als Wundauflage gemäß einem der vorstehenden Ansprüche, wobei die Wundauflage in einer Vorrichtung zur Unterdrucktherapie von Wunden umfassend
(a) ein Abdeckmaterial zum luftdichten Verschließen eines Wundraums;
(b) gegebenenfalls ein Mittel zum Anschließen einer Unterdruckquelle; und
(c) die Wundauflage
enthaltend ist.
